# EUROPEAN PATENT APPLICATION

(11) **EP 2 156 821 A1**
(43) Date of publication of application: **24.02.2010**
(21) Application number: 08752700.8
(22) Date of filing: 14.05.2008
(51) Int. Cl.: A61K 8/88, A61K 8/34, A61K 8/37, A61K 8/891, A61Q 1/02, A61Q 1/04, A61Q 1/08, A61Q 1/10, A61Q 15/00

(54) **OIL-BASED SOLID COSMETIC**

(30) Priority: 15.05.2007 JP 2007129085
(71) Applicant: Kokyu Alcohol Kogyo Co., Ltd., Narita-shi Chiba 287-0225 (JP)
(72) Inventor: YOSHIDA, Mari, Narita-shi Chiba 287-0225 (JP); SATO, Nobumasa, Yokohama-shi Kanagawa 236-0004 (JP); KOKEGUCHI, Yuki, Narita-shi Chiba 287-0225 (JP); SONE, Hidetoshi, Narita-shi Chiba 287-0225 (JP)
(74) Representative: Nuss, Laurent
(86) International application number: PCT/JP2008/058829
(87) International publication number: WO 2008/143081

(57) **Abstract**

Disclosed is an oil-based solid cosmetic which has superior shaping property, skin-repairing effect, skin-moisturizing effect and long-lasting make-up effect to those of a conventional one. Specifically disclosed is an oil-based cosmetic comprising a polyamide resin, a branched alcohol and a dihydric alcohol.

## Description

### Technical Field

The present invention relates to an oily solid cosmetic **characterized in that** it comprises a polyamide resin, in particular an amide-terminated polyamide resin and/or an ester-terminated polyamide resin, a branched alcohol and a divalent alcohol.

### Background Art

In the prior art oily solid cosmetic, waxes have been blended in a high amount as the simple method for increasing cosmetic persistence. However, as the adverse effects thereof, there is a problem that preferable qualities, such as skin repair effect and skin moisturizing property must be sacrificed.

On the other hand, solidifying agents used for cosmetics include those using polyamide resins, in addition to waxes that are commonly used. For example, in Patent Publication 1, the structured composition comprising a liquid fat phase that is structured by a polymer bonded to an amphiphilic compound having a certain HLB value is described, wherein according to such a composition, liquid oil can be formed into a stick form without wax, and the composition can be structured without opacifying it by the above polymers.

Patent Publication 2 describes the composition that is a solid form comprising liquid continuous fat phase structured by polyamide resin and not comprising wax, wherein coloring material, liquid fat phase and polymer construct a physiologically acceptable medium.

In addition, Patent Publication 3 describes that the composition comprising a polyamide resin, diisostearyl malate, polyglyceryl diisostearate-2 and liquid oil is utilized as the oily solid cosmetics such as lipstick, hair stick, hand stick, etc.. Further, this publication also describes the oily solid cosmetic comprising a polyamide resin, ethylhexyl hydroxystearate (IOHS) which is a 12-hydroxystearate ester.

Furthermore, Patent Publication 6 describes the stick comprising a certain polyamide resin, 2-octyl dodecanol and 2-hexyl decyl alcohol or 1,3-butyleneglycol.

However, these oily solid cosmetics using polyamide resin do not always have sufficient skin repair effect, skin moisturizing effect and cosmetic persistence.
Therefore, development of the technologies and products for materializing an oily solid cosmetic that accomplishes these inconsistentqualities,namely,skinrepaireffect,skinmoisturizing effect as well as cosmetic persistence at the same time is strongly required.
[Patent Publication 1] Patent No. 3466138
[Patent Publication 2] Patent No. 3609010
[Patent Publication 3] U.S. Patent Publication No.2006/0280763
[Patent Publication 4] JP, A, 2002-534535
[Patent Publication 5] JP, A, 2002-516619
[Patent Publication 6] JP, B, 7-98731

### Disclosure of Invention

### Problems to be Solved by the Invention

Therefore, an object of the present invention is to provide an oily solid cosmetic having superior skin repair effect, skin moisturizing effect and cosmetic persistence as compared to conventional products.

### Means for Solving the Problems

Considering the above problems, the inventors have sought an ingredient having a comparable properties to those of waxes which increase cosmetic persistence and possibly being blended in a large amount, an ingredient which maintains and increases skin repair effect, skin moisturizing effect and cosmetic persistence, and in addition a combination thereof being free from problems in productivity, stability, etc. Surprisingly, the inventors found out during the research that, by using a specific alcohol together with a polyamide resin having high solidification ability as well as high elasticity, not only the above performances of the polyamide resin and cosmetic persistence of the cosmetic is maintained and increased, but also skin repair effect and skin moisturizing effect are increased, having completed the present invention after further intensive research.

Thus, the present invention relates to an oily solid cosmetic comprising a polyamide resin, a branched alcohol and a divalent alcohol.
Further, the present invention relates to the oily solid cosmetic, wherein the polyamide resin is one or more selected from the following amide-terminated polyamide resin: 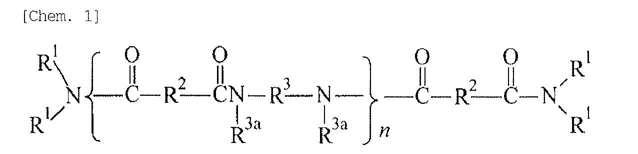 wherein R¹ is a C₈ to C₂₂ alkyl group which may be the same or different with each other, R² is a dimer acid residue, R³ is an ethylenediamine residue, R^{3a} is hydrogen, and n=2 to 4,
and the following ester-terminated polyamide resin: 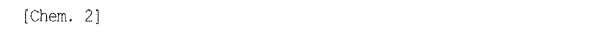 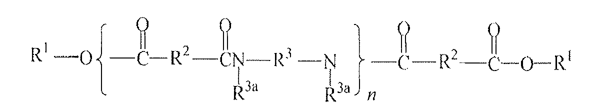 wherein R¹, R², R³, R^{3a} and n are as defined above.

Furthermore, the present invention relates to the oily solid cosmetic wherein the branched alcohol is one or more selected from hexyl decanol, isostearyl alcohol, octyl dodecanol, decyl tetradecanol, dodecyl hexadecanol, tetradecyl octadecanol, and hexadecyl eicosanol.
Furthermore, the present invention relates to the oily solid cosmetic wherein the divalent alcohol is one or moreselected from 1,3-butyleneglycol, pentyleneglycol, propyleneglycol, dipropylene glycol, hexylene glycol, octylene glycol, and polypropylene glycol having the average molecular weight of 200 to 2000.
Furthermore, the present invention relates to the oily solid cosmetic further comprising a 12-hydroxystearate ester.
Still further, the present invention relates to the oily solid cosmetic further comprising a lubricant.
Further, the present invention relates to the oily solid cosmetic further comprising silicone oil.
Further, the present invention relates to the oily solid cosmetic further comprising thickening agent and/or a gelling agent.
Furthermore, the present invention relates to the oily solid cosmetic, wherein the silicone oil is methylphenyl polysiloxane and/or dimethyl polysiloxane.
Further, the present invention relates to a process for increasing cosmetic persistence of an oily solid cosmetic comprising a polyamide resin, which comprises a step of adding a branched alcohol and a divalent alcohol to the oily solid cosmetic.

As discussed above, the oily solid cosmetic of the present invention satisfies skin repair effect, skin moisturizing effect and cosmetic persistence at the same time, that are inconsistent qualities and could not be accomplished by the conventional products, by comprising a branched alcohol and a divalent alcohol together with a polyamide resin.
According to the present invention, since the polyamide resin has cosmetic persistence as well as properties similar to those of waxes, it facilitates formation of a cosmetic, being possibly blended in a large amount.
Further, whereas both the branched alcohol and the divalent alcohol are used as a moisturizing ingredient or a solvent in the cosmetic, both of them contribute to increase or at least maintain not only skin moisturizing effect, but also cosmetic persistence and skin repair effect according to the present invention.
Furthermore, none of these ingredients shows any problems in productivity, stability, etc. of the ingredient itself and the cosmetic, and combined use of these ingredients synergistically increases the effects of each ingredient.

### Effects of the Invention

The oily solid cosmetic of the present invention has the effects that all of skin repair effect, skin moisturizing effect and cosmetic persistence are excellent.
Further, the oily solid cosmetic of the present invention wherein the polyamide resin is one or more selected from the above-described amide-terminated polyamide resin and/or ester-terminated polyamide resin achieves particularly excellent skin repair effect, skin moisturizing effect and cosmetic persistence effect
Further, the oily solid cosmetic of the present invention wherein the branched alcohol is one or more selected from hexyl decanol, isostearyl alcohol, octyl dodecanol, decyl tetradecanol, dodecyl hexadecanol, tetradecyl octadecanol and hexadecyl eicosanol, or the one wherein the divalent alcohol is one or more selected from 1,3-butyleneglycol, pentylene glycol,propylene glycol, dipropylene glycol, hexylene glycol, octylene glycol and polypropylene glycol having the average molecular weight of 200 to 2000 achieves particularly excellent skin repair effect, skin moisturizing effect and cosmetic persistence effect.
Further, the oily solid cosmetic of the present invention further comprising a 12-hydroxystearate ester achieves particularly excellent skin repair effect, skin moisturizing effect and cosmetic persistence effect.
Furthermore, the oily solid cosmetic of the present invention further comprising a lubricant achieves further excellent solidification properties and the feel as well as skin moisturizing effect of the cosmetic.
The oily solid cosmetic of the present invention further comprising silicone oil achieves further excellent feel and skin moisturizing effect of the cosmetic.
The oily solid cosmetic of the present invention further comprising a thickening agent and/or a gelling agent achieves further excellent solid nature of the cosmetic.
The oily solid cosmetic of the present invention wherein the silicone oil is methylphenyl polysiloxane and/or dimethyl polysiloxane achieves further excellent skin moisturizing effect of the cosmetic.
According to the process for increasing the cosmetic persistence of the oily solid cosmetic comprising a polyamide resin of the present invention, the cosmetic persistence of the oily solid cosmetic can be increased without impairing skin repair effect and skin moisturizing effect by a simple method such as adding a branched alcohol and a divalent alcohol to the oily solid cosmetic.

### Best Mode for Carrying Out the Invention

The present invention is hereinafter explained in further detail.
Meanwhile, in the present specification, "solidification property", "skin repair effect", "skin moisturizing effect" and "cosmetic persistence" have the meanings as follows respectively.
"Solidification property": a property or degree capable of having the desired solid or semi-solid shape etc.
"Skin repair effect": an effect of preventing or improving skin irritation, which may correspond to treatment effect.
"Skin moisturizing effect": an effect of inhibiting evaporation of moisture from skin, which may correspond to moisture preserving effect.
"cosmetic persistence": an effect of persisting the desired effect by a makeup, which may correspond to long lasting effect.
Furthermore, in the present specification, "solid cosmetic" is a material which can retain a solid shape at normal temperature (about 25 °C) possibly used for cosmetics such as lipstick, lip cream, foundations, eye colors, blushes, eyebrows, deodorant agents, fragrance hair sticks or hand sticks.
Furthermore, "increasing" the cosmetic persistence etc. by adding a certain ingredient to a cosmetic means that the cosmetic persistence etc. of the cosmetic comprising such ingredient is superior to the cosmetic persistence etc. of the cosmetic free from such ingredient.

The polyamide resin used in the present invention is specifically not limited; an amide-terminated polyamide resin and/or an ester-terminated polyamide resin represented by the following formulae are preferable.
Amide-terminated polyamide resin 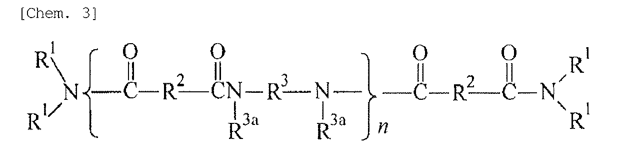 Ester-terminated polyamide resin 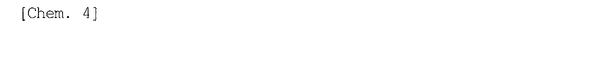 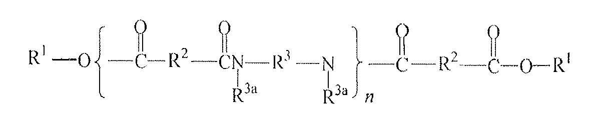 wherein R¹ is a C₈ to C₂₂ alkyl group which may be the same or different with each other, R² is a dimer acid residue, R³ is an ethylenediamine residue, R^{3a} is hydrogen, and n=2 to 4.
In the above formulae, R¹ may be a straight-chain or a branched-chain alkyl group; as the straight-chain alkyl group, C₈: octyl group, C₁₀: decyl group, C₁₂: lauryl group, C₁₄: myristyl group, C₁₆: palmityl group, C₁₈: stearyl group, C₂₀: arachidyl group, and C₂₂: behenyl group are exemplified. As a branched-chain alkyl group, C₈: 2-ethylhexyl group, C₉: isononyl group, C₁₀: isodecyl group, C₁₃: isotridecyl group, and C₁₈: isostearyl group are exemplified. Of these, C₈ to C₂₀ alkyl group is preferable, C₁₄ to C₂₀ alkyl group is more preferable, and C₁₄ to C₁₈ alkyl group is the most preferable.
In the above formulae, as R², dimer dilinoleic acid residue which is a dimeric acid residue, and the residues of adipic acid, pimelic acid, suberic acid, azelaic acid, sebacic acid, undecane dioic acid, dodecane dioic acid, tridecane dioic acid, tetradecane dioic acid, pentadecane dioic acid, octadecane dioic acid, nonadecane dioic acid and eicosane dioic acid that are dibasic acids are exemplified. Among these examples , dimer dilinoleic acid residue is the most preferable.

As the examples of the above preferable polyamide resin, SYLVACLEAR A200V, A2614V as the amide-terminated polyamide resin and UNICLEAR 100VG as the example of the ester-terminated polyamide resin are exemplified, but the polyamide resin used in the present invention is not limited thereto.
Meanwhile, detail descriptions can be found regarding the amide-terminated polyamide resin and the ester-terminated polyamide resin in Patent Publication 4 and 5, respectively.
The amount of the polyamide resin is not limited;, it is preferably 5 to 70 mass %, more preferably 10 to 65 mass %, most preferably 15 to 63 mass % of the entire oily solid cosmetic from the viewpoint of solidification property.
The amide-terminated polyamide resin can consist of (a-1) high viscosity amide-terminated polyamide resin and (a-2) low viscosity amide-terminated polyamide resin. The amount of (a-1) is preferably 3 to 40 mass %, more preferably 5 to 30 mass %, most preferably 10 to 20 mass % of the total amount of the amide-terminated polyamide resin. Theamountof (a-2) is preferably 4 to 40 mass%, more preferably 5 to 35 mass %, most preferably 15 to 25 mass %. The total amount of (a-1) and (a-2) is adjusted according to the type and content of the other ingredients and a variety of properties desired in the solid cosmetic.

The branched alcohols used in the present invention is not specifically limited; the alcohols with 10 to 40 carbon atoms are preferably used. As the branched alcohols preferably used in the present invention, hexyl decanol, isostearyl alcohol, octyl dodecanol, decyl tetradecanol, dodecyl hexadecanol, tetradecyl octadecanol, and hexadecyl eicosanol are exemplified. As to the branched alcohols, one kind may be used, and plural kinds may also be used in combination. Further, though the amount of the branched alcohols used in the present invention is not specifically limited as well, 0.1 to 20 mass % of the entire oily solid cosmetic is preferable, 0.3 to 15 mass % is more preferable, and 0.5 to 12 mass % is most preferable from the viewpoint of maintenance and increase of skin repair effect, skin moisturizing effect, and cosmetic persistence.

The divalent alcohols used in the present invention is not specifically limited; 1,3-butylene glycol, pentylene glycol, propylene glycol, dipropylene glycol, hexylene glycol, octylene glycol, and polypropylene glycol having the average molecular weight of 200 to 2000, etc. are preferably used. As the divalent alcohol, one of the above alcohols may be used, and plurality thereof may also be used in combination.
Further, the amount of the divalent alcohols used in the present invention is not specifically limited as well, 0.01 to 10 mass % of the entire oily solid cosmetic is preferable, 0.03 to 7 mass % is more preferable, and 0.05 to 5 mass % is most preferable from the viewpoint of maintenance and increase of skin repair effect, skin moisturizing effect, and cosmetic persistence.

A 12-hydroxystearate ester used in the present invention is also a known material and not specifically limited; the ester with alcohol having 1 to 30 carbon atoms is preferable. More specific examples of preferable esters include ethylhexyl hydroxystearate, octyl hydroxystearate, phytosteryl hydroxystearate, cholesteryl hydroxystearate, etc.
Furthermore, the amount of the 12-hydroxystearate esters used in the present invention is not specifically limited as well; 7 to 70 mass % of the entire oily solid cosmetic is preferable, 10 to 65 mass % is more preferable, 12 to 63 mass % is even more preferable, and 24 to 60 mass % is most preferable from the viewpoint of maintenance and increase of skin repair effect, skin moisturizing effect, and cosmetic persistence.
The oily solid cosmetic of the present invention using a 12-hydroxystearate ester is preferable because of an advantage of particularly excellent skin repair effect, skin moisturizing effect and effect of cosmetic persistence.

The lubricant used in the present invention is not specifically limited, animal and vegetable fats and hydrogenated animal and vegetable fats such as avocado oil, linseed oil, almond oil, privet wax, nettle tree oil, olive oil, cacao fat, kapok wax, Japanese nutmeg oil, carnauba wax, liver oil, candelilla wax, beef fat, beef leg gat, beef bone fat, hardened beef fat, apricot kernel oil, whale wax, hardened oil, wheat germ oil, sesame oil, rice germ oil, rice bran oil, sugarcane wax, sasanqua oil, safflower oil, shea butter, Paulownia fagesii oil, cinnamon oil, jojoba wax, shellac wax, turtle oil, soybean oil, tea berry oil, camellia oil, evening primrose oil, cone oil, lard, rapeseed oil, Japanese empress tree oil, bran wax, germ oil, horse fat, persic oil, palm oil, palm core oil, castor oil, hardened castor oil, castor oil fatty acid methyl ester, sunflower oil, grapefruit oil, bay berry wax, jojoba oil, macadamia nut oil, beeswax, mink oil, cottonseed oil, cotton wax, Japan wax, Japan wax core oil, montan wax, palm tree oil, hardened palm tree oil, tri-palm oil fatty acid glyceride, mutton tallow, earthnut oil, lanolin, liquid lanolin, reduced lanolin, lanolin alcohol, hard lanolin, lanolin acetate, lanolin fatty acid isopropyl, hexyl laurate, POE lanolin alcohol ether, POE lanolin alcohol acetate, lanolin fatty acid polyethyleneglycol, POE hydrogenated lanolin alcohol ether, egg-yolk oil, etc.;
hydrocarbon oils, such as ozokerite, squalane, squalene, ceresin, paraffin, paraffin wax, liquid paraffin, pristane, polyisobutylene, microcrystalline wax, Vaseline, etc.;
higher fatty acids, such as lauric acid, myristic acid, palmitic acid, stearic acid, behenic acid, undecylenoic acid, oleic acid, linoleic acid, linolenic acid, arachidonic acid, eicosapentaenoic acid (EPA), docosahexaenoic acid (DIIA), isostearic acid, 12-hydroxystearic acid, etc.;
higher alcohols, such as lauryl alcohol, myristyl alcohol, palmityl alcohol, stearyl alcohol, behenyl alcohol, hexadecyl alcohol, oleyl alcohol, cetostearyl alcohol, cholesterol, phytosterol, POE cholesterol ether, monostearyl glycerin ether (batyl alcohol), monooleyl glyceryl ether (selachyl alcohol), etc.;
ester oils, such as, isononyl isononanoate, isotridecyl isononanoate, etc. that are isononanoate esters, cetyl ethylhexanoate, hexyldecyl ethylhexanoate, etc. that are 2-ethylhexanoate esters, isopropyl myristate, isocetyl myristate, octyldodecyl myristate, etc. that are myristate esters, ethyl isostearate, isopropyl isostearate, hexyldecyl isostearate, isostearyl isostearate, etc. that are isostearate esters, isostearyl lactate, ocryldodecyl lactate, etc. that are lactate esters, ethylhexyl hydroxystearate, octyl hydroxystearate, phytosteryl hydroxystearate, cholesteryl hydroxystearate, etc. that are hydroxystearate esters, oleyl oleate, phytosteryloleate, octyldodecyl oleate, etc., that are oleate esters, isodecyl neopentanoate, isostearyl neopentanoate, etc. that are neopentanoate esters, isopropyl palmitate, ethylhexyl palmitate, etc. that are palmitate esters, in addition to these, octyldodecyl ricinoleate, octyldodecyl erucate, etc. for monoesters,
diisobutyl adipate, diisopropyl adipate, diethylhexyl succinate, neopentyl glycol diethylhexanoate, neopentyl glycol dicaprate, diisostearyl malate, diisopropyl dilinoleate, ethyleneglycol dioctanoate, octyldodecyl stearoyloxystearate, diisopropyl sebacate, etc. for diesters,
triethylhexanoin, trimethylolpropane triethylhexanoate, glycerin tri (caprylate/caprate), trimethylolpropane triisostearate, erythrityl triethylhexanoate, etc. for trimesters,
pentaerythrityl tetraethylhexanoate, pentaerythrityl tetraisostearate, etc. for tetraesters,
polyglyceryl isostearate-2, polyglyceryl diisostearate-2, etc. that are polyglycerin fatty acid esters for polyesters;
high viscosity ester lubricant, such as (hydroxystearic acid/stearic acid/resin acid) dipentaerythrityl, isostearic acid hydrogenated castor oil, dimer dilinoleic acid hydrogenated castor oil, (polyglyceryl isostearate-2/dimer dilinoleic acid) copolymer, dimer dilinoleic acid (phytosteryl/isostearyl/cetyl/stearyl/behenyl), dimer dilinoleic acid dimer dilinoleyl bis(phytosteryl/behenyl/isostearyl), dimer dilinoleic acid di(isostearyl/phytosteryl), dimer dilinoleyl hydrogenated rosin condensate, dimer dilinoleyl diisostearate, dimer dilinoleyl dimer dilinoleic acid, etc. are exemplified. Furthermore, any other lubricants other than the above can be used as long as encompassed as those accomplishing the object of the present invention.

The amount of the lubricant used in the present invention is not specifically limited as well; from the viewpoint of solidification property, the amount is preferably 1 to 15 mass % of the entire oily solid cosmetic, more preferably 2 to 12 mass %, and most preferably 5 to 10 mass %.

The oily solid cosmetic of the present invention further comprising a silicone oil or a thickening agent and/or a gelling agent as other ingredients is preferable from the viewpoint of solidification property and skin moisturizing property.
The silicone oil used in the present invention is not specifically limited; silicone compounds, such as dimethyl polysiloxane, methylphenyl polysiloxane, alkyl denatured organopolysiloxane, terminal denatured organopolysiloxane, fluorine denatured organopolysiloxane, amodimethicone, amino denatured organopolysiloxane, volatile silicone, alkyldimethicone, etc. are exemplified, methylphenyl polysiloxane and dimethyl polysiloxane with excellent general versatility can be used preferably.
The amount of the silicone oil used in the present invention is not specifically limited as well; from the viewpoint of solidification property and skin moisturizing property, the amount is preferably 1 to 20 mass % of the entire oily solid cosmetic, more preferably 3 to 15 mass %, and most preferably 5 to 10 mass %.

The thickening agent and the gelling agent used in the present invention are not specifically limited as well; natural and artificial thickening agents or gelling agents, such as alginic acid, polyaspartic acid, deoxyribo nucleic acid and salts thereof, guar gum, agar-agar, gelatin, sodium polyacrylate, cellulose ester, calcium alginate, carboxyvinyl polymer, ethylene/acrylic acid copolymer, vinylpyrrolidone polymer, vinyl alcohol/vinylpyrrolidone copolymer, nitrogen substituted acrylamide polymer, polyacrylamide, cationized guar gum, dimethylacrylammonium polymer, acrylic acid-acryl metacrylate copolymer, POE/POP copolymer, polyvinyl alcohol, pullulan, tamarind seedpolysaccharides, xanthangum, carrageenan, high methoxyl pectin, low methoxyl pectin, gum arabic, crystalline cellulose, arabinogalactan, karayagum, tragacanthgum, albumin, casein, curdlan, gellan gum, dextrin fatty acid ester, cellulose, polyethyleneimine, highly polymerized polyethylene glycol, cationized silicone polymer, synthetic latex, C18 or higher alkyl dimethicone or silicone polyamide copolymer that is a silicon gelling agent, N-2-ethyl hexanoyl-L-glutamic acid dibutyl amide, N-lauroyl-L-glutamic acid dibutyl amide, (behenic acid eicosandioic acid)glyceryl, (behenic acid eicosandioic acid)polyglyceryl-10 etc. are exemplified.

Furthermore, in the oily solid cosmetic of the present invention, various ingredients can be used such as organic ultraviolet screening agents, lubricants, thickening gelling agents, surfactants, antiseptic agents, antimicrobials, fragrances, humectants, salts, solvents, antioxidants, chelating agents, neutralizing agents, pH regulators, insect rejectants, bioactive ingredients, etc., that are usually used in the oily cosmetic.
As the bioactive ingredient used in the present invention, materials giving certain bioactivities to skin when applied to skin are exemplified. For example, anti-inflammatory agents, antiaging agents, tightening agents, antioxidative agents, humectants, blood circulation accelerators, antimicrobials, microbicides, desiccants, cool sense agents, warm sense agents, vitamins, amino acids, wound healing accelerators, torpents, analgesics, cell activator agents, enzyme ingredients, etc. are exemplified. According to the present invention, one or more of these bioactive ingredients are preferably blended.

As the form of the oily solid cosmetic of the present invention, for example, lipstick, lip cream, foundation, eye color, blush, deodorant agent, fragrance hair stick or hand stick, etc. are exemplified.

The process for increasing the cosmetic persistence of the oily solid cosmetic according to the present invention comprising a polyamide resin comprises a step of adding a branched alcohol and a divalent alcohol to the oily solid cosmetic; the addition of the branched alcohol and the divalent alcohol to the oily solid cosmetic may be carried out either at the same time as the addition of the polyamide resin, or before or after the addition of the polyamide resin. Thus, according to the above-described method of the present invention, cosmetic persistence as well as skin repair effect and skin moisturizing effect of the oily solid cosmetic can be increased by comprising a branched alcohol and a divalent alcohol eventually.
The present invention is hereinafter further specifically explained by means of Examples and Comparative examples relating to the oily solid cosmetic of the present invention, but the present invention should not be limited by any way by these Examples.
Furthermore, unless specified otherwise, "%" described below means "mass %".

### (Evaluation of availability)

### [Method for preparing samples]

The samples for each test were prepared by shaping by a conventional method and allowing to stand for 24 hours at 25 °C.
In addition, the formulations of each Example and Comparative example (lipstick, blush, foundation, eye color and deodorant agent) were as shown in series in Tables 2, 4, 6, 8 and 10.
Hereinbelow, the polyamide resins used in each Example were amide-terminated polyamide resins.

### [Method for evaluating availability]

Twenty female panelists from20s to 40s were selected at random, and evaluations were carried out as to skin repair effect, skin moisturizing effect and cosmetic persistence. The criteria of evaluations were as shown in Table 1 below. Furthermore, the evaluation results of each Example and Comparative Example whose formulations were as shown in series in Tables 2, 4, 6, 8 and 10 were as shown in series in Tables 3, 5, 7, 9 and 11, respectively.

[Table 1]

**Table 1:**

| The number of person who answered as "good" of 20 | Evaluation |
|---|---|
| 15 or more | ⊚ |
| 10 to 14 | ○ |
| 5 to 9 | Δ |
| 0 to 4 | × |

[Table 2]

**Table 2: Formulation (lipstick)**

| Name of ingredient | Exemplary example | | |
|---|---|---|---|
| | 1 | 2 | 3 |
| Polyamide resin | 20.0 | 30.0 | 40.0 |
| Ceresin (hydrocarbon oil) | | | |
| Polyethylene | | | |
| Octyl dodecanol | | | 5.0 |
| Isostearyl alcohol | 5.0 | 7.0 | 5.0 |
| 1.3-Butylene glycol | 1.0 | 1.0 | 1.0 |
| Dipropylene glycol | 1.0 | 1.0 | 1.0 |
| Propylene glycol | 0.5 | 0.5 | 0.5 |
| Ethylhexyl hydroxystearate | to. 100 | to. 100 | to. 100 |
| Diisostearyl malate | 5.0 | 5.0 | 5.0 |
| Hydrogenated polyisobutene | 5.0 | 5.0 | 5.0 |
| Polyglyceryl diisostearate-2 | 5.0 | 5.0 | 5.0 |
| Red No. 201 | 0.2 | 0.2 | 0.2 |
| Red No. 202 | 0.1 | 0.1 | 0.1 |
| Yellow iron oxide | 0.5 | 0.5 | 0.5 |
| Black iron oxide | 0.1 | 0.1 | 0.1 |
| Titanium oxide | 2.0 | 2.0 | 2.0 |
| Mica titanium | 8.0 | 8.0 | 8.0 |
| Total | 100.0 | 100.0 | 100.0 |
| | | | |

| Comparative example | | | |
|---|---|---|---|
| 1 | 2 | 3 | 4 |
| | | | |
| 20.0 | 40.0 | | |
| | | 20.0 | 40.0 |
| | | | |
| | | | |
| | | | |
| | | | |
| | | | |
| to. 100 | to. 100 | to. 100 | to. 100 |
| 5.0 | 5.0 | 5.0 | 5.0 |
| 5.0 | 5.0 | 5.0 | 5.0 |
| 5.0 | 5.0 | 5.0 | 5.0 |
| 0.2 | 0.2 | 0.2 | 0.2 |
| 0.1 | 0.1 | 0.1 | 0.1 |
| 0.5 | 0.5 | 0.5 | 0.5 |
| 0.1 | 0.1 | 0.1 | 0.1 |
| 2.0 | 2.0 | 2.0 | 2.0 |
| 8.0 | 8.0 | 8.0 | 8.0 |
| 100.0 | 100.0 | 100.0 | 100.0 |

[Table 3]

**Table 3: Evaluation results (lipstick)**

| Evaluation results | Exemplary example | | | Comparative example | | | |
|---|---|---|---|---|---|---|---|
| | 1 | 2 | 3 | 1 | 2 | 3 | 4 |
| Skin repair effect | ⊚ | ⊚ | ⊚ | Δ | × | Δ | × |
| Skin moisturizing effect | ⊚ | ○ | ○ | × | × | × | × |
| Cosmetic persistence | ⊚ | ⊚ | ⊚ | Δ | × | Δ | × |

[Table 4]

**Table 4: Formulation (blush)**

| Name of ingredient | Exemplary example | | |
|---|---|---|---|
| | 4 | 5 | 6 |
| Polyamide resin | 20.0 | 30.0 | 40.0 |
| Ceresin | | | |
| Polyethylene | | | |
| Decyl tetradecanol | | 3.0 | |
| Isostearyl alcohol | 5.0 | 4.0 | 10.0 |
| 1,3-Butylene glycol | 0.5 | 0.5 | 1.0 |
| Dipropylene glycol | 0.5 | 0.5 | 0.5 |
| Ethylhexyl hydroxystearate | to. 100 | to. 100 | to. 100 |
| Diisostearyl malate | 7.0 | 7.0 | 7.0 |
| Polyglyceryl diisostearate-2 | 2.0 | 2.0 | 2.0 |
| Red No. 226 | 0.1 | 0.1 | 0.1 |
| Yellow iron oxide | 1.0 | 1.0 | 1.0 |
| Ultramarine blue | 0.1 | 0.1 | 0.1 |
| Titanium oxide | 2.0 | 2.0 | 2.0 |
| Mica titanium | 3.0 | 3.0 | 3.0 |
| Total | 100.0 | 100.0 | 100.0 |
| | | | |

| Comparative example | | | |
|---|---|---|---|
| 5 | 6 | 7 | 8 |
| | | | |
| 20.0 | 40.0 | | |
| | | 20.0 | 40.0 |
| | | | |
| | | | |
| | | | |
| | | | |
| to. 100 | to. 100 | to. 100 | to. 100 |
| 7.0 | 7.0 | 7.0 | 7.0 |
| 2.0 | 2.0 | 2.0 | 2.0 |
| 0.1 | 0.1 | 0.1 | 0.1 |
| 1.0 | 1.0 | 1.0 | 1.0 |
| 0.1 | 0.1 | 0.1 | 0.1 |
| 2.0 | 2.0 | 2.0 | 2.0 |
| 3.0 | 3.0 | 3.0 | 3.0 |
| 100.0 | 100.0 | 100.0 | 100.0 |

[Table 5]

**Table 5: Evaluation results (blush)**

| Evaluation results | Exemplary example | | | Comparative example | | | |
|---|---|---|---|---|---|---|---|
| | 4 | 5 | 6 | 5 | 6 | 7 | 8 |
| Skin repair effect | ⊚ | ⊚ | ○ | Δ | Δ | Δ | Δ |
| Skin moisturizing effect | ⊚ | ○ | ○ | × | × | × | × |
| Cosmetic persistence | ⊚ | ⊚ | ⊚ | Δ | × | Δ | × |

[Table 6]

**Table 6: Formulation (foundation)**

| Name of ingredient | Exemplary example | | |
|---|---|---|---|
| | 7 | 8 | 9 |
| Polyamide resin | 30.0 | 40.0 | 50.0 |
| Ceresin | | | |
| Polyethylene | | | |
| Hexyl decanol | 5.0 | | |
| Isostearyl alcohol | | 7.0 | 10.0 |
| 1,3-Butylene glycol | 0.5 | 0.7 | 1.0 |
| Dipropylene glycol | 0.5 | 0.5 | 0.5 |
| Ethylhexylhydroxystearate | to. 100 | to. 100 | to. 100 |
| Isononyl isononanoate | 3.0 | 3.0 | 3.0 |
| Methyl polysiloxane | 2.0 | 2.0 | 2.0 |
| Colcothar | 0.4 | 0.4 | 0.4 |
| Yellow iron oxide | 0.8 | 0.8 | 0.8 |
| Ultramarine blue | 0.1 | 0.1 | 0.1 |
| Titanium oxide | 10.0 | 10.0 | 10.0 |
| Total | 100.0 | 100.0 | 100.0 |
| | | | |

| Comparative example | | | |
|---|---|---|---|
| 9 | 10 | 11 | 12 |
| | | | |
| 30.0 | 50.0 | | |
| | | 30.0 | 50.0 |
| | | | |
| | | | |
| | | | |
| | | | |
| to. 100 | to. 100 | to. 100 | to. 100 |
| 3.0 | 3.0 | 3.0 | 3.0 |
| 2.0 | 2.0 | 2.0 | 2.0 |
| 0.4 | 0.4 | 0.4 | 0.4 |
| 0.8 | 0.8 | 0.8 | 0.8 |
| 0.1 | 0.1 | 0.1 | 0.1 |
| 10.0 | 10.0 | 10.0 | 10.0 |
| 100.0 | 100.0 | 100.0 | 100.0 |

[Table 7]

**Table 7: Evaluation results (foundation)**

| Evaluation results | Exemplary example | | | Comparative example | | | |
|---|---|---|---|---|---|---|---|
| | 7 | 8 | 9 | 9 | 10 | 11 | 12 |
| Skin repair effect | ⊚ | ⊚ | ○ | Δ | Δ | Δ | Δ |
| Skin moisturizing effect | ○ | ○ | ○ | × | × | × | × |
| Cosmetic persistence | ⊚ | ⊚ | ⊚ | × | × | × | × |

[Table 8]

**Table 8: Example (eye color)**

| Name of ingredient | Exemplary example | | |
|---|---|---|---|
| | 10 | 11 | 12 |
| Polyamide resin | 20.0 | 30.0 | 40.0 |
| Ceresin | | | |
| Polyethylene | | | |
| Isostearyl alcohol | 5.0 | 7.0 | 10.0 |
| 1,3-Butylene glycol | 1.0 | 1.5 | 2.0 |
| Ethylhexyl hydroxystearate | to. 100 | to. 100 | to. 100 |
| Liquid paraffin | 8.0 | 8.0 | 8.0 |
| Methylphenyl polysiloxane | 3.0 | 3.0 | 3.0 |
| Colcothar | 0.1 | 0.1 | 0.1 |
| Yellow iron oxide | 0.3 | 0.3 | 0.3 |
| Ultramarine blue | 0.1 | 0.1 | 0.1 |
| Red No. 226 | 0.1 | 0.1 | 0.1 |
| Mica titanium | 10.0 | 10.0 | 10.0 |
| Total | 100.0 | 100.0 | 100.0 |
| | | | |

| Comparative example | | | |
|---|---|---|---|
| 13 | 14 | 15 | 16 |
| | | | |
| 20.0 | 40.0 | | |
| | | 20.0 | 40.0 |
| | | | |
| | | | |
| to. 100 | to. 100 | to. 100 | to. 100 |
| 8.0 | 8.0 | 8.0 | 8.0 |
| 3.0 | 3.0 | 3.0 | 3.0 |
| 0.1 | 0.1 | 0.1 | 0.1 |
| 0.3 | 0.3 | 0.3 | 0.3 |
| 0.1 | 0.1 | 0.1 | 0.1 |
| 0.1 | 0.1 | 0.1 | 0.1 |
| 10.0 | 10.0 | 10.0 | 10.0 |
| 100.0 | 100.0 | 100.0 | 100.0 |

[Table 9]

**Table 9: Evaluation results (eve color)**

| Evaluation results | Exemplary example | | | Comparative example | | | |
|---|---|---|---|---|---|---|---|
| | 10 | 11 | 12 | 13 | 14 | 15 | 16 |
| Skin repair effect | ⊚ | ⊚ | ○ | Δ | Δ | Δ | Δ |
| Skin moisturizing effect | ⊚ | ○ | ○ | × | × | × | × |
| Cosmetic persistence | ⊚ | ⊚ | ⊚ | Δ | × | Δ | × |

[Table 10]

**Table 10: Formulation (deodorant agent)**

| Name of ingredient | Exemplary example | | |
|---|---|---|---|
| | 13 | 14 | 15 |
| Polyamide resin | 40.0 | 50.0 | 60.0 |
| Ceresin | | | |
| Polyethylene | | | |
| Dodecyl hexadecanol | | | 1.5 |
| Isostearyl alcohol | 1.0 | 2.0 | 1.5 |
| 1,3-Butylene glycol | 0.5 | 0.5 | 0.5 |
| Ethylhexyl hydroxystearate | to. 100 | to. 100 | to. 100 |
| Diisostearyl malate | 3.0 | 3.0 | 3.0 |
| Methylphenyl polysiloxane | 15.0 | 15.0 | 15.0 |
| Methyl polysiloxane | 2.0 | 2.0 | 2.0 |
| Fragrance | 3.0 | 3.0 | 3.0 |
| Total | 100.0 | 100.0 | 100.0 |
| | | | |

| Comparative example | | | |
|---|---|---|---|
| 17 | 18 | 19 | 20 |
| | | | |
| 40.0 | 60.0 | | |
| | | 40.0 | 60.0 |
| | | | |
| | | | |
| | | | |
| To. 100 | to. 100 | to. 100 | to. 100 |
| 3.0 | 3.0 | 3.0 | 3.0 |
| 15.0 | 15.0 | 15.0 | 15.0 |
| 2.0 | 2.0 | 2.0 | 2.0 |
| 3.0 | 3.0 | 3.0 | 3.0 |
| 100.0 | 100.0 | 100.0 | 100.0 |

[Table 11]

**Table 11: Evaluation results (deodorant agent)**

| Evaluation results | Exemplary example | | | Comparative example | | | |
|---|---|---|---|---|---|---|---|
| | 13 | 14 | 15 | 17 | 18 | 19 | 20 |
| Skin repair effect | ⊚ | ○ | ○ | Δ | × | Δ | × |
| Skin moisturizing effect | ⊚ | ⊚ | ○ | × | × | × | × |
| Cosmetic persistence | ⊚ | ○ | ○ | Δ | × | Δ | × |

As shown above, it was established that the oily solid cosmetic of the present invention has the effect of cosmetic persistence superior to the one using waxes, can be blended in a high amount, and in addition has excellent performances in maintaining and increasing skin repair effect, skin moisturizing effect and cosmetic persistence.

### (Evaluation of solidification property etc.)

### [Samples]

The compositions consisting of the following ingredients described in Patent Publication 1 and Patent Publication 2 as Exemplary examples (Comparative example 21) and (Comparative example 22) were prepared respectively, and evaluations of solidification property, extensibility and adherability were carried out.

[Table 12]

**Table 12: lipstick**

| Name of ingredient | Comparative example 21 |
|---|---|
| UNICLEAR 80 | 25.0 |
| Parleam oil | 56.0 |
| Polyglyceryl-2 polyhydroxystearate | 10.0 |
| Pigment | 9.0 |
| Total | 100.0 |

[Table 13]

**Table 13: anhydrous blush**

| Name of ingredient | Comparative example 22 |
|---|---|
| UNICLEAR 80 | 25.0 |
| Parleam oil | 35.1 |
| Glyceryl oleate | 31.25 |
| Pigment | to. 100.0 |
| Total | 100.0 |

### [Results]

Each of the above compositions, being free from a branched alcohol and a divalent alcohol in contrast to the cosmetic of the present invention, showed a paste form, being difficult to be shaped into a lipstick. Furthermore, both shaped products of lipstick and blush had poor extensibility, and the feeling thereof was inferior to the cosmetic of the present invention.

The composition consisting of the following ingredients described in Patent Publication 3 as the Example (Comparative example 23) was prepared, and evaluated its extensibility and adherability.

[Table 14]

**Table 14: lipstick**

| Name of ingredient | Comparative example 23 |
|---|---|
| Polyamide resin | 22.0 |
| Diisostearyl malate | 15.0 |
| Polyglyceryl-2 diisostearate | 40.0 |
| Isotridecyl isononanoate | 23.0 |
| Total | 100.0 |

### [Results]

The above composition, also free from a branched alcohol and a divalent alcohol in contrast to the cosmetic of the present invention, showed inferior feeling such as extensibility and adherability to that of the cosmetic of the present invention.

### [Industrial Applicability]

According to the present invention, the oily solid cosmetic having superior skin repair effect, skin moisturizing effect and cosmetic persistence to the prior art products is provided. Therefore, the present invention greatly contributes to the development of cosmetic industry and related industries.

## Claims

1. An oily solid cosmetic comprising a polyamide resin, a branched alcohol and a divalent alcohol.

2. The oily solid cosmetic according to Claim 1, whereon the polyamide resin is one or more selected from the following amide-terminated polyamide resin: 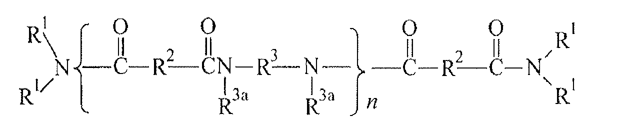 wherein R¹ is a C₈ to C₂₂ alkyl group which may be the same or different with each other, R² is a dimer acid residue, R³ is an ethylenediamine residue, R^{3a} is hydrogen, and n=2 to 4,
and the following ester-terminated polyamide resin: 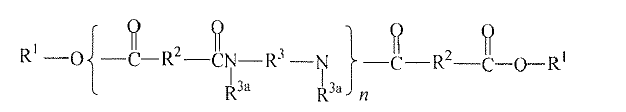 wherein R¹, R², R³, R^{3a} and n are as defined above.

3. The oily solid cosmetic according to Claims 1 or 2, wherein the branched alcohol is one or more selected from hexyl decanol, isostearyl alcohol, octyl dodecanol, decyl tetradecanol, dodecyl hexadecanol, tetradecyl octadecanol, and hexadecyl eicosanol.

4. The oily solid cosmetic according to any one of Claims 1 to 3, wherein the divalent alcohol is one or more selected from 1,3-butylene glycol, pentylene glycol, propylene glycol, dipropylene glycol, hexylene glycol, octylene glycol, and polypropylene glycol having the average molecular weight of 200 to 2000.

5. The oily solid cosmetic according to any one of Claims 1 to 4, further comprising a 12-hydroxystearate ester.

6. The oily solid cosmetic according to any one of Claims 1 to 5, further comprising a lubricant.

7. The oily solid cosmetic according to any one of Claims 1 to 6, further comprising silicone oil.

8. The oily solid cosmetic according to any one of Claims 1 to 6, further comprising a thickening agent and/or a gelling agent.

9. The oily solid cosmetic according to Claim 7, wherein the silicone oil is methylphenyl polysiloxane and/or dimethyl polysiloxane.

10. A process for increasing cosmetic persistence of an oily solid cosmetic comprising a polyamide resin, which comprises a step of adding a branched alcohol and a divalent alcohol to the oily solid cosmetic.
